(19)

Europäisches
Patentamt
European
Patent Office
Office européen
des brevets

(11) **EP 3 771 446 A1**

(12) **EUROPEAN PATENT APPLICATION**

(43) Date of publication:
**03.02.2021  Bulletin 2021/05**

(21) Application number: **19189197.7**

(22) Date of filing: **30.07.2019**

(51) Int Cl.:
**A61B 17/34** (2006.01)    **A61B 34/30** (2016.01)
**A61B 90/50** (2016.01)    **A61F 9/007** (2006.01)
**B25J 1/00** (2006.01)    **B25J 18/00** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Katholieke Universiteit Leuven
KU Leuven Research & Development
3000 Leuven (BE)**

(72) Inventors:
• **SMITS, Jonas
3360 Korbeek-Lo (BE)**
• **VANDER POORTEN, Emmanuel
2800 Mechelen (BE)**

(54)    **MECHANICAL REMOTE MOTION CONSTRAINING MECHANISM**

(57)    A system for constraining remote motion of a robot or mechanism in an environment is described. It comprises a robot or a mechanism subsystem for establishing remote motion of an instrument or of a remote portion with a number of degrees of freedom with respect to an object and an alignment subsystem, coupled to said robotic subsystem or mechanism, for providing additional degrees of freedom for moving the robotic subsystem or mechanism with respect to the object of interest. The system furthermore comprises a fixturing means comprising a first rigid body or set of first rigid bodies with known first shape being adjustably fixed to a moveable part of the alignment subsystem, and a second rigid body or second set of rigid bodies with known second shape being adjustably fixed to an environment. The first rigid body or set of rigid bodies and the second rigid body or set of rigid bodies are shaped and positioned with respect to each other so that contact between the first rigid body or set of rigid bodies and the second rigid body or sets of rigid bodies provide restriction to the remote movement of the instrument, of the movement of a remote portion of the system or of the object.

**FIG. 5**

**Description**

**Field of the invention**

The invention relates to the field of mechanics or mechatronics. More specifically it relates to methods and systems for assisting in positioning a robot or a mechanism and/or for constraining remote motion of the robot or the mechanism by a mechanical proximal constraining means.

**Background of the invention**

During robot-assisted surgery, robots or mechanisms operate in close vicinity with humans to handle tissue, muscles, organs and so on. Often a robot is deployed such as to improve the precision in e.g. removing, dissecting or suturing tissue. Mostly the robotic system or mechanism is steered such as to handle the diseased or damaged tissue whereas healthy tissue, vessels, and so on or to be left intact as much as possible. Researchers have been implementing software based means, so-called 'active constraints' or 'virtual fixtures' to prevent a robot for entering zones where tissue, nerve, muscle or organ damage would occur. Other researchers developed passive guides or semi-active guides whereby a number of Degrees of Motion are selectively locked to prevent motion in a certain direction or entry in a certain region. The basic idea consists of dynamically constraining, at each instant, the direction of possible motions of each joint, depending on the task to be performed. A computer computes which joints needs to be locked and which needs to be kept free to avoid certain regions. A passive movable frame was developed that is mounted distally at the patient to physically restrain instrument motion in a conical area that was needed for the targeted procedure. While such frame provides excellent and stable guidance, due to size constraints it is often impossible to build up such distal reference system. Furthermore this approach is not very well suited for building up complex geometric constraints. To allow rigid and stable constraints, yet avoid space occupancy in the vicinity of the patient one could get inspiration from remote center of motion mechanisms (RCMs).

During robot-assisted surgery, a Remote Center of Motion (RCM) is often implemented to constrain instrument motion through and about a specific point in space. A mechanical structure, a so-called RCM mechanism, is then able to transfer pivotal motion about a Local Center of Motion (LCM) to a distal location. An instrument held by an RCM mechanism will then be constrained to perform pivotal motion about a distal pivot point (the RCM).

Insertion/retraction of the instrument through that same distal point is often possible as well. When implemented in a back-drivable mechanism, the instrument can then be operated intuitively so that it is unhindered in all DoFs (Degrees-of-Freedom) that are not constrained and prevented to move in all constrained DoFs, here thus resulting in pivotal motion.

In various applications it is desirable to allow a similar intuitive and unhindered motion relative to another higher-dimensional constrained geometry such as a line, a curvilinear path, a plane or more generic a 3-dimensional surface. It would be advantageous if such constraints would be adequately aligned with a distal (anatomical) structure such as to prevent distal motion violating said constraints. Furthermore, it would be particularly advantageous if such distal constraints could be implemented relying on proximal mechanical means rather than on distal mechanical means such that distal space occupancy can be kept minimal.

One example where such property would be for example of interest is in aligning of RCM mechanisms themselves. As mentioned before an RCM mechanism limits distal motion to move about a distal RCM point. An alignment system is often used to locate this distal RCM point in space mostly in line with some anatomical reference. If the alignment mechanism could be constrained such that alignment itself is constrained to specific regions in space one could guarantee that during the RCM alignment phase itself safety constraints could be adhered to. This particular example is discussed further in greater detail. Another example not related to RCM alignment mechanisms is presented afterwards.

The kinematic architecture of surgical robotic systems can often be generalized as a combination of two subsystems. Firstly, a "RCM subsystem" provides 1 translational (T) and 2 rotational (R) Degrees of Freedom (DoFs) of the surgical instrument through and about a defined RCM. Secondly, an "Alignment subsystem" provides additional DoFs to move the RCM subsystem with respect to the patient, thus enabling alignment of the RCM with the point of entry in the patient's body. In Minimally Invasive Surgery (MIS) long and slender instruments are often used that access the patient through one or more incisions or keyholes. A surgical port or cannula is then commonly inserted in the incision to guide the instrument while protecting the tissue at the incision from damage due to repeated relative motion between instrument and tissue. The incision and surgical port form here an artificially created point of entry. In other minimal invasive procedures natural orifices are used to access the body and reach the patient's internals.

An advantage of systems with a RCM is that, when precisely aligned with the surgical entry point, stresses to surrounding tissue can be kept to a minimum.

While the RCM functionality can be advantageous when properly aligned with the surgical entry point, it is not always trivial to do so as this is often a non-visualized point in space. The same holds for the point of entry in the patient's body. The incision or the natural orifice that serves as a point of entry is a volume rather than a single identifiable point. Researchers have identified an optimal pivot point (OPP) as a central point within this volume that would lead to minimal stresses on surrounding tissue and developed strategies to identify such OPP. When misalignment between the RCM and the OPP occurs, the RCM might lead to elevated tissue stresses, thus defeating its original purpose. Furthermore, during procedures where the RCM requires repositioning during surgery, proper alignment is highly challenging. This might generally occur due to unforeseen patient movement, due to variations in the patient's anatomy, variable operating parameters (e.g. variable insufflation pressure), but may also be intentional to improve the reachability or visualization during a procedure such as natural orifice surgery or intra-ocular eye surgery. Not being able to safely and intuitively reposition the RCM for these purposes, limits the usage of surgical robotics. This is further detailed in the following for the use cases of vitreoretinal surgery, laparoscopic surgery and natural orifice surgery.

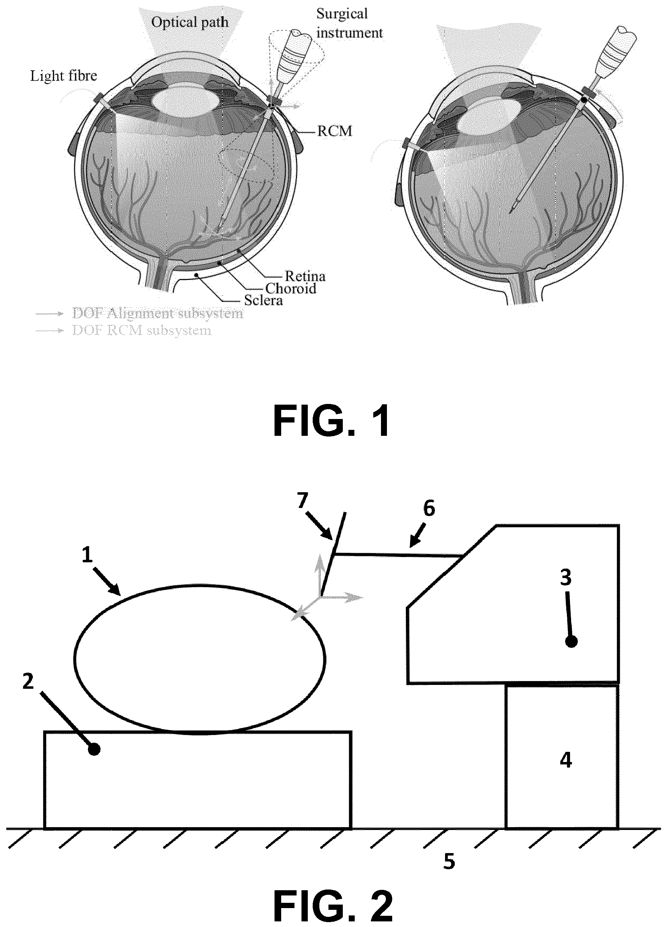

Vitreoretinal surgery is a branch of microsurgery involving delicate interactions with the inner and posterior segments of the eye (Fig. 1). Surgical scene visualisation is done looking through the eye lens, using a surgical microscope and additional corrective optics. Procedures are highly challenging, and limited by human factors such as hand tremor and lack of depth/force perception. Surgical robotic technology and smart instrumentation can offer increased precision and situational awareness. Robotic assistance can be achieved with either a teleoperative approach using motion-scaling or a cooperative approach using force-scaling. The interested reader is referred to Molaei et al. in Journal of Ophthalmic & Vision Research 12 (2017) pp 212-218 for an overview of eye-surgical robotic systems, and to Taylor in the International Journal of Robotics Research 18 (1999) pp1201 - 1210 for information on cooperative eye-surgical robots. First applications of robot-assisted eye surgery have been successfully demonstrated during first-in-human clinical studies on retinal vein cannulation as described by Gijbels et al. in Annals of Biomedical Engineering 46 (2018) pp 1676-1685 and epiretinal membrane peeling as described by Edwards et al. in Nature Biomedical Engineering 2 (2018) September. During robotic eye surgery, the RCM is aligned with a small incision in the eye wall, or sclera, through which the surgical instrument is inserted. Accurate positioning of the RCM is important from both clinical as well as a usability perspective. From a clinical perspective, excess scleral forces due to RCM misalignment could cause physiological complications such as increased scarring and retinal detachment. From a usability perspective, misalignment of the RCM with the incision point can cause unwanted eye rotations when moving the instrument, resulting in posterior target anatomy to move towards or away from the instrument tip. During high-accuracy procedures such as retinal vein cannulation and subretinal injections, this raises surgical complexity and risk. Surgical scene visualisation is also affected, as the microscope's optical path changes due to eye lens movements. During procedures such as pars plana vitrectomy or epiretinal membrane peeling, surgeons may wish to adjust the position of the eye intra-operatively. During manual surgery the surgeons use the inserted instruments as levers to intentionally rotate the eye in order to visualize other segments of the retina (Fig. 1). In a scenario where surgical robots with an RCM are employed, this would require repositioning of the RCM to another acceptable location. During such adjustment the RCM should preferably coincide with the moving surgical port. Since the eye is spherical the port would rotate about the eye's centre. For the RCM to follow with minimal stress on the eye wall, the RCM should therefore also move along the surface of the eye globe together with the instrument port. Without any form of guidance steering a multi-DoF device along a specific surface is not straightforward. For these reasons it is believed that improving surgical port alignment accuracy through the usage of a guidance method can reduce both risk of complications and surgical complexity during vitreoretinal surgery.

In laparoscopic surgery surgeons make several incisions in the patient's abdomen to introduce a camera (laparoscope) and one or more instruments such as grasper's, cautery, suction or other tools. Thanks to the smaller scars the patient trauma is reduced and recovery can be faster. Manipulating of the plurality of instruments through these small incisions is generally more difficult. Long learning curves (often exceeding 100 procedures) to master this interventions are therefore quite common. Some of the appeal for robotic approaches stems from their intuitiveness in use and the relatively shorter associated learning curves. While simplifying task execution, commercial surgical robotic devices do not offer haptic feedback. Therefore surgeons often lack proper understanding of the governing interaction forces. Within the camera field of view surgeons typically infer the interaction forces from the deformations that are taking place. Relying on their expertise and knowledge of the response of anatomical structures they can gauge the applied force levels. However, outside the camera's view robotic surgeons are practically 'blind' and have little notice of the size of the forces that are being applied. The incision where the robotic instruments enter the patient's body in keyhole surgery is typically such a location that is out of view. For robots with an RCM-mechanism the purpose of an RCM alignment mechanism is to minimize the interaction forces at the level of the incision. However, even if perfectly aligned, e.g. coincident with an OPP, in the beginning, over time the incision may shift due to e.g. removal of tissue, physiological processes, leakage or buildup of internal pressure. This explains the increased level of trocar-side hernia (TSH) that has been observed in such robotic laparoscopic procedures. A guidance mechanism that would dynamically follow the motion of the body wall and in response would assist the medical staff in re-positioning the robot's RCM - e.g. by means of a dynamic fixture that follows the observed motion - could protect the body wall from RCM-induced tissue damage.

In natural orifice surgery, one of the body's natural orifices is used to get access to the patient's internals. Long slender instruments are employed to reach deeply seated structures. Depending on the type of procedure these instruments can be amongst others catheters, guidewires, needles, rigid or flexible scopes. When these instruments are held or driven by a robotic structure alignment is necessary to position the instruments in front of the natural orifice. This alignment most commonly involves placing the instrument roughly at the center of the orifice and orienting it such that it is aligned more or less along the orifice's longitudinal direction. Since mostly the orifice diameter is larger than the instrument diameter there is often some more freedom in positioning. E.g. one may want to position off-centre or take on another orientation such as to reach specific deeply located points. When deviating too much from the centre or when overly inclining the instrument the latter could make intense contact with the orifice's surrounding tissues which could also lead to undesirable tissue damage (or even damage to the instrument). Thus also in the case of natural orifice surgery a system that assists in keeping the RCM point within the boundaries of the orifice would be desirable. Such system could then e.g. constrain the motion of the alignment system to remain in line with the anatomical features.

Different methods have been reported to provide intraoperative positioning guidance of the RCM during robot-assisted surgery. The RCM can be visualized, for instance using a specific joint configuration or by laser projection. While sufficient for initial alignment, RCM repositioning requires the surgeon to shift gaze away from the surgical scene and towards the surgical port. This negatively affects surgical risk, usability, and time. Force sensing methods have been proposed in order to reduce tissue stress surrounding the surgical port. In the field of eye surgery, related work has addressed the concern of excessive scleral forces. The proposed method relies on actively measuring the scleral interaction forces, which are used to actively reposition the robot in order to reduce scleral interaction forces. However, this solution relies on the usage of fragile force-sensing instruments and costly measurement devices. Furthermore, its good functioning is based on active control relying on a dynamic model and a control law limited to admittance-type control schemes. Positional guidance in robot assisted surgery can also be achieved using haptic fixtures, and is a well-documented research topic as described by Abbott et al. in Robotics Research Berlin, Heidelberg: Springer Berlin Heidelberg (2007) pp 49-64. Haptic fixtures can be viewed as a specific geometry aiding the user with their robotic positioning task. At least the following two types of fixtures are of interest in robot assisted surgery: 1) forbidden-region fixtures (FRF), which prevent the user from moving into the fixture geometry; 2) Guidance fixtures (GF), which aid the user in tracking the fixture surface geometry. Even though haptic fixtures in robot-assisted surgery are commonplace as a research topic, efforts tend to be focussed on preventing instrument tip interactions with the surgical scene. In its simplest form, a FRF can be implemented based on a local distance estimation between instrument and anatomy as described in Balicki et al. in Medical Image Computing and Computer-Assisted Intervention MICCAI (2009) Berlin, Heidelberg : Springer pp 108-115. No further information on surrounding anatomy is required. Another approach is to use anatomical information to generate the fixture geometry. Such approaches have been reported for robot-assisted knee surgery as described by Davies et al. in Proceedings of the Institution of Mechanical Engineers, part H : Journal of Engineering in Medicine 211 (1997) pp 285-292 and nasal surgery as described in Li et al. in IEEE Transactions on Robotics 23 (2007) pp 4 - 19. To the author's best knowledge, only a single report has been made on the usage of guidance fixtures to address the problem of intraoperative RCM positioning during robot-assisted eye surgery. Nasseri et al. reported in the 5th IEEE RAS/EMBS International Conference on Biomedical Robots and Biomechatronics, IEEE (2014) pp732-738, on a virtual fixture control method for RCM placement using a teleoperatively controlled setup for eye surgery. The setup requires such a solution, as it does not rely on a hardware-based RCM. In general, virtual fixture implementations offer high geometric flexibility, yet add complexity and stability considerations, and thus surgical risk. Furthermore, fixtures for RCM positioning will most likely need to restrict motion of larger payloads when compared to fixtures for instruments, which might require a higher level of stiffness. It is believed that fixtures for RCM positioning might not require the same degree of geometric complexity and flexibility, and could therefore benefit from a less flexible, yet less complex, hardware-based alternative. Such an alternative would especially offer additional value for impedance-type devices which excel in transparency and simplicity, yet are limited in rendering stiff fixtures compared to admittance devices.

Also in other applications that are not necessarily related to aligning an RCM, but more generally in positioning a distal instrument relative with respect to the patient's anatomy, it is desirable to allow a similar intuitive and unhindered motion relative to higher-dimensional constrained geometries. For example to replace the weight-bearing surfaces of the knee joint to relieve pain and disability during Total Knee Arthroplasty, the ends of the distal end of the femur and the proximal end of the tibia need to be accurately cut to shape. While it is an option to use cutting guides that are oriented along the long axis of the bones, using proximal guides to constrain the distal motion clear up the area at the operating site and possibly reduce the risk for infection. If used in combination with a back-drivable system and a proximal mechanism for constraining the distal motion, the operator could freely and confidently move the cutter in the unconstrained region assured that the instrument will not protrude into the constrained region.

## Summary of the invention

It is an object of embodiments of the present invention to provide safe and reliable methods and systems for assisting in positioning a robot or mechanism and/or for constraining remote motion of the mechanism or robot by a mechanical proximal constraining means.

It is an advantage of embodiments of the present invention that safe and reliable methods and systems for RCM positioning by a mechanical proximally located constraining means are provided.

It is an advantage of embodiments of the present invention that methods and systems are provided for constraining distal motion using anatomy-based haptic fixtures.

It is an advantage of embodiments of the present invention that methods and systems are provided for RCM positioning using anatomy-based haptic fixtures.

It is an advantage of embodiments of the present invention that methods and systems are provided enabling safe, high-stiffness guidance fixtures.

It is an advantage of at least some embodiments of the present invention that underdamped responses due to fixture interaction, e.g. unexpected fixture behaviours, are avoided since these could result in potentially harmful instrument motions.

It is an advantage of at least some embodiments of the present invention that the fixture can be implemented in addition to other haptic features, such as tremor reduction or fixtures for the instrument tip. It is an advantage of embodiments of the present invention that fixture interaction does not negatively affect the performance other system features, e.g. surgical system features.

It is an advantage of embodiments of the present invention that a simplified positioning with respect to a distal constraining geometry is obtained.

It is an advantage of embodiments of the present invention that a simplified alignment of RCM points is obtained.

It is an advantage of embodiments of the present invention that an instrument held by a robot or mechanism can be constrained to a set of locations which are in line with some anatomic features.

It is an advantage of embodiments of the present invention that the RCM can be constrained to a set of locations which are in line with some anatomic features.

It is an advantage of embodiments of the present invention that these sets of locations could be pre-defined and in line with some anatomic features.

It is an advantage of embodiments of the present invention that these pre-defined sets of locations can be updated intra-operatively e.g. based on sensory data or instructions from medical staff.

It is an advantage of embodiments of the present invention that an instrument held by a robot or mechanism can operated faster and/or more precise relative to a set of constraints.

It is an advantage of embodiments of the present invention that faster and/or more precise alignment of the RCM can be obtained.

It is an advantage of embodiments of the present invention that these provide enhanced safety guarantees and simplifies intraoperative adjustments of the constraint locations during the intervention.

It is an advantage of embodiments of the present invention that these provide enhanced safety guarantees and simplifies intraoperative adjustments of RCM locations, i.e. adjustments of the RCM point during the intervention.

It is an advantage of embodiments of the present invention that the system allows for deriving an optimal positioning of the remote center of motion. For example, an optimal pivot point, can be defined as a location in the body wall that induces minimal stress when an instrument is pivoting around this point. In a static scenario, using embodiments of the present invention, this point could be determined once and kept stationary. Nevertheless, embodiments of the present invention also allow for adjustments during intervention, e.g. surgical intervention. For example, during an intervention the body wall may change position e.g. due to changes in intra-operative pressure or due to physiological motion. Embodiments of the present invention allow for example to take into account the movement of the body wall and e.g. actively adjust the pivot point motion in par with such body wall motion. This is advantageous and can for example further limit the invasiveness of the procedure and potentially reduce the occurrence of TSH.

It is an advantage of at least some embodiments according to the present invention that exertion of forces towards the patient during repositioning can be avoided. This enhances the usability of the embodiments according to the present invention.

It is an advantage of at least some embodiments according to the present invention that the introduction of risks, e.g. surgical risks, introduced by actively rendering patient-oriented forces can be reduced.

It is an advantage of at least some embodiments according to the present invention that good accuracy can be obtained by avoiding positional deadband between different force fields. Fully virtual implementations of multiple force fields in near vicinity of each other as well as surface tracking fixtures often require the implantation of a positional deadband in order to be used in a safe and stable manner. Not doing so can give rise to potentially unstable limit cycling behaviour around the fixture transition areas, which in turn elevates surgical risk. Furthermore, this deadband causes a loss in fixture accuracy. Mechanical or hybrid fixture implementations are able to reduce or eliminate the occurrence of limit cycling behaviour without the need of additional deadband.

It is an advantage of embodiments of the present invention that a guidance feature with higher stiffness is obtained, and thus a guidance that provides improved surface tracking accuracy. The present invention relates to a system for assisting in positioning an instrument or a remote portion of the system relative to a working environment or to a system for assisting in constraining remote motion of a subsystem or of an instrument in a working environment, the system comprising,

a robotic subsystem or mechanism for positioning the instrument with one or more degrees of freedom with respect to an object,

an alignment subsystem, coupled to said robotic subsystem or mechanism, for providing one or more degrees of freedom for moving the robotic subsystem or mechanism and/or the instrument with respect to the object of interest,

the system further comprising a fixturing means, the fixturing means comprising a first body with known first shape or a plurality of first bodies with known first shapes, referred to as the probe, the probe being adjustably fixed to a moveable part of the alignment subsystem, and

a single second body with known second shape or a plurality of second bodies with known second shapes, referred to as the probed surface, the probed surface being adjustably fixed to the environment wherein the robotic subsystem or mechanism is operating,

whereby the probe and the probe surface are shaped and positioned with respect to each other so that contact between the probe and the probed surface provide restriction of the movement of the robotic subsystem or mechanism and/or of the instrument.

According to embodiments of the present invention, the system may be used for assisting in constraining an instrument, e.g. a medical instrument, or a remote portion of the system relative to the working environment. It is to be noted that the system already may be used usefully by merely constraining the movement of a remote portion of the system, even when no instrument is present. In the case of an RCM mechanism, the remote portion of the system corresponds with the RCM mechanism that is typically used for holding the instrument. Where in embodiments reference is made to a robotic subsystem, reference is made to a set of linkages that can be actuated by actuators. Where reference is made to a mechanism, reference is made to linkages, but these do not necessarily need to be actuated but can be purely passive.

Where reference is made to the probe being adjustably fixed to a moveable part of the alignment subsystem, reference is made to the fact that a position and/or an orientation of the first body is adjustably fixed with respect to the alignment subsystem, or with respect to a reference frame linked, e.g. fixed, to the alignment subsystem. The second body may be referred to as the probe surface. Where reference is made to the second body being adjustably fixed to the environment, reference is made to a position and/or orientation of the second body being fixed to the environment or with respect to a reference frame linked, e.g. fixed, to the environment.

Where in embodiments of the present invention reference is made to "adjustably fixed", reference is made to the fact that the operator may first adjust the position, the orientation or even the shape of the body e.g. based on anatomic data of the object, based on the operation room layout, based on personal preferences, and that the body thereafter is fixed, typically prior to the start of the operation.

The robotic subsystem or mechanism may contain or may be a remote center of motion (RCM) mechanism. The remote portion of the system that is constrained may be the remote center of motion point. The contact between the probe and the probed surface may provide restriction to the movement of the remote center of motion point.

The shape of the probe and/or the shape of the probed surface may be determined as function of the anatomy of the object.

The probe and/or the probed surface may be rigid bodies.

The position of the probe and/or the position of the probed surface may be determined as function of the anatomy of the object.

The fixturing means may be configured for keeping the probe and he probed surface into contact with each other for defining a mechanical restriction for the instrument movement or the movement of the remote portion of the system.

The alignment subsystem may be rigidly coupled to the robotic subsystem or mechanism. The fixturing means may be configured for keeping the probe and the probed surface into contact with each other for defining a mechanical restriction for the instrument motion or for the movement of the remote portion of the system, e.g. an RCM.

The system furthermore may be configured for providing a virtual force field that attracts, repels, slows down or stabilizes the motion of the instrument, of the robotic subsystem or mechanism or of the movement of a remote center of motion.

The system may be configured to estimate the state or the alteration of the state of a remote object and may alter the position of the probe and / or the position of the probed surface in response to measured state or the alteration of the state of the remote object.

The position of the probe and the probed surface may be altered in a coordinated fashion such as to advantageously program the shape of the region where instrument movement or movement of a remote center of motion is restricted.

The present invention also relates to a system for assisting in positioning of a remote center of motion system in an environment, the system comprising,

a remote center of motion subsystem for establishing a remote center of motion with one or more degrees of freedom to an instrument with respect to an object,

an alignment subsystem, coupled to said remote center subsystem, for providing one or more degrees of freedom for moving the remote center of motion subsystem with respect to the object of interest,

the system further comprising a fixturing means, the fixturing means comprising a first body with known first shape, referred to as the probe, the first body being adjustably fixed to a moveable part of the alignment subsystem, and

a second body with known second shape, referred to as the probed surface, the second body being adjustably fixed to the environment wherein the remote center of motion mechanism of the robot is operating,

whereby the first body and the second body are shaped and positioned with respect to each other so that contact between the first body and the second body provide restriction of the RCM movement.

The first body may be a rigid bogy. The second body may be a rigid body.

The first body may be referred to as the probe. Where reference is made to the probe being adjustably fixed to a moveable part of the alignment subsystem, reference is made to the fact that a position and/or an orientation of the first body is adjustably fixed with respect to the alignment subsystem, or with respect to a reference frame linked, e.g. fixed, to the alignment subsystem. The second body may be referred to as the probe surface. Where reference is made to the second body being adjustably fixed to the environment, reference is made to a position and/or orientation of the second body being fixed to the environment or with respect to a reference frame linked, e.g. fixed, to the environment.

Where in embodiments of the present invention reference is made to "adjustably fixed", reference is made to the fact that the operator may first adjust the position, the orientation or even the shape of the body e.g. based on anatomic data of the object, based on the operation room layout, based on personal preferences, and that the body thereafter is fixed, typically prior to the start of the operation.

The contact between the first and the second body may define a sliding interface that determines a surface geometry with respect to the object, e.g. patient, or a reference system linked thereto, the surface geometry being defined by the position and shape of the first and second body. This surface geometry defines advantageous positions of the RCM with respect to the object, e.g. patient, or the reference system linked thereto.

The material of the first and the second body may be selected such as to create a certain desirable sliding behavior e.g. due to specific static and/or dynamic friction coefficients governing the frictional sliding interaction between the first and the second body. One or more of the first and second body may be made out of ferromagnetic material and foreseen of an electromagnet such that upon magnetizing the body attraction force can be generated between the bodies and the relative motion of one body with respect to the other body can be altered. In some embodiments gross motion may be even prevented upon activating the electromagnets or gross motion may be enabled upon activating the electromagnets.

It is an advantage of embodiments of the present invention that the system does not require patient-oriented forces to be rendered.

It is an advantage of embodiments of the present invention that the positional accuracy can be high, due to the high restriction stiffness based on the mechanical fixturing means. It is an advantage of embodiments of the present invention that the positioning is inherently safe since the restrictions are hardware based, thus preventing safety issued that may arise when applying (or applying only) software-based restrictions, e.g. fully based on virtual forces applied with impedance-controlled robots.

The shape of the first body and/or the shape of the second body may be determined as function of the anatomy of the object.

The shape may be determined as function of an average anatomy of a particular position on the object that is subject to surgery, especially in cases where the deviation from this average geometry does not induce overly large stresses or strains onto the targeted anatomy, although in some embodiments the shape may be defined as function of a specific patient anatomy. The position of the first body and/or the position of the second body may be determined as function of the anatomy of the object. The position may be determined as function of an average anatomy of a particular position on the object that is subject to surgery, although in some embodiments the shape may be define as function of a specific patient anatomy.

The fixturing means may be configured for keeping the first and the second body into contact with each other for defining a mechanical restriction for the RCM movement.

In any of the above aspects, also the following features may be present or applied.

The alignment subsystem may be rigidly coupled to the remote center subsystem.

It is an advantage of embodiments of the present invention that such a rigid coupling can be used for positioning the RCM and the alignment subsystem (and thus also the first body) with respect to the object, e.g. patient.

The second body or probed surface may be fixed to the environment via a further alignment subsystem, allowing controlled positioning of the second body or probed surface with respect to the first body or probe. It is an advantage of embodiments of the present invention that the second body or probed surface can be positioned independently with respect to the first body or probe.

The further alignment subsystem may provide the same degrees of freedom as the alignment subsystem to which the first body or probe is adjustably fixed.

The first body and the second body or the probe and probe surface, when into contact, may form any of a sphere on a sphere, a sphere on a plate or surface, a sphere in a hollow cylinder or a cylinder on a plate or surface. The system furthermore may be configured for providing a virtual attractive force field to the remote center of motion.

The system may be configured for providing a virtual attractive force field for repositioning the remote center of motion along a surface defined by different positions of the first body or probe and the second body or probe surface when the first body or probe and the second body or probe surface are in contact with each other.

It is an advantage of embodiments of the present invention that the operator is aided in tracking the remote center of motion along the surface defining the optimum locations for the remote center of motion. It is an advantage of embodiments of the present invention that the remote center of motion can be repositioned while moving the instrument away from the patient simultaneously, thus preventing the need for a user to keep applying a force to maintain a contact between the first and the second body or probe and probed surface in order to keep the RCM stationary or to move the RCM dynamically along the interface between the first and the second body or probe and probed surface. It is an advantage of embodiments of the present invention that the restriction of movement of the remote center of motion can be performed using a fixturing means rendering the restriction automated rather than to be applied by the operator based on information to be captured by the operator from the environment at the moment of repositioning.

The system may be configured for allowing repositioning of the robotic subsystem or mechanism during an intervention, e.g. a surgical intervention.

The present invention also relates to a robot for use in an environment, the robot comprising a system for assisting in positioning of a robotic subsystem or mechanism or the robot comprising a system for assisting in positioning of a remote instrument or of a remote portion of the system or of a remote center of motion of the robot as described above. The robot may be a back-drivable robot

The present invention furthermore relates to a method for assisting in positioning of a robotic subsystem in an environment, the method comprising positioning a robotic subsystem with respect to an object by controlling positioning of the robotic subsystem using a system as described above. It also relates to a method for assisting in positioning of a remote center of motion system in an environment, the method comprising positioning a remote center of motion with respect to an object by controlling positioning of the remote center of motion using a system as described above. The method may comprise estimating the shape of a remote object, the shape of the anatomy o location of a feature of the object or the anatomy based upon which the probe and the probe surface can be computed, defined or positioned. The fixture shape may be created using a system as described above.

In these methods, the environment may be an operating theater.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

## Brief description of the drawings

FIG. 1 shows an annotated illustration of a surgical scene using a 3R1T+3T surgical robotic system architecture with RCM depicting the instrument mobility offered by an RCM subsystem DoFs and by an RCM alignment subsystem.

FIG. 2 illustrates a generalized surgical scene using a robotic subsystem with a robotic end-effector holding a surgical instrument, depicting a possible instrument mobility offered by the robotic subsystem's DoFs and/or by the robotic subsystem's alignment subsystem.

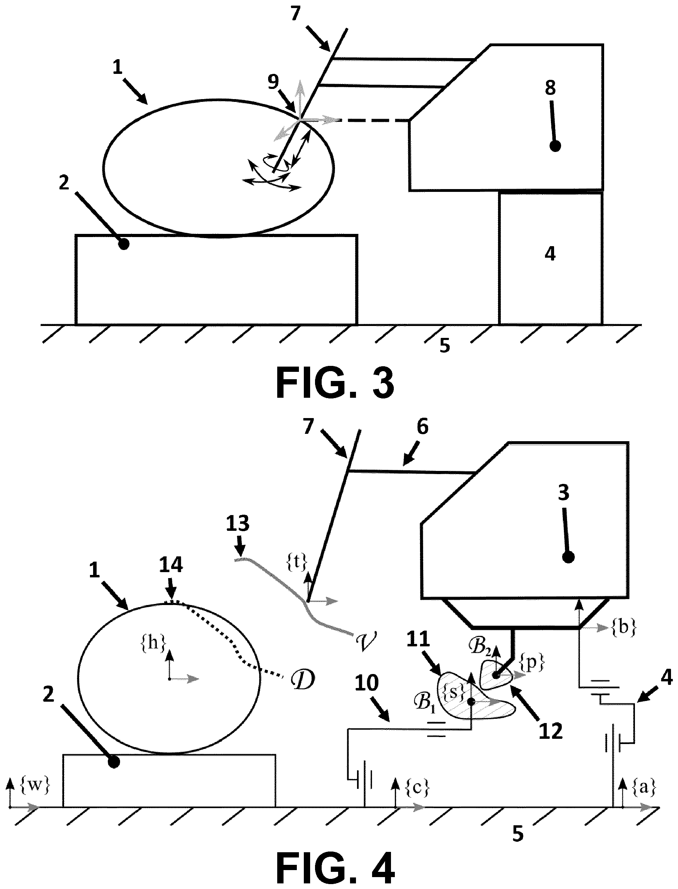

FIG. 3 illustrates a generalized surgical scene using a 3R1T+3T surgical robotic system architecture with an RCM depicting the instrument mobility offered by an RCM subsystem DoFs and by an RCM alignment subsystem.

FIG. 4 illustrates a generalized surgical scene using a surgical robotic subsystem (3) in which embodiments of the present invention can be used. Reference coordinate frames: {a} alignment subsystem base, {b} robotic subsystem base, {t} instrument tip, {h} patient, {w} world, {c} fixture mechanism, {s} a first constraining $B_1$, {p} a second constraining $B_2$. The alignment of the robotic subsystem is constrained by a fixture V defined in the world frame.. The constraining fixture could be aligned with e.g. a group of admissible instrument tip locations D based on a patient's anatomy by appropriately positioning the constraining body 1. Note that V and D thereby are virtual elements represented in the drawing

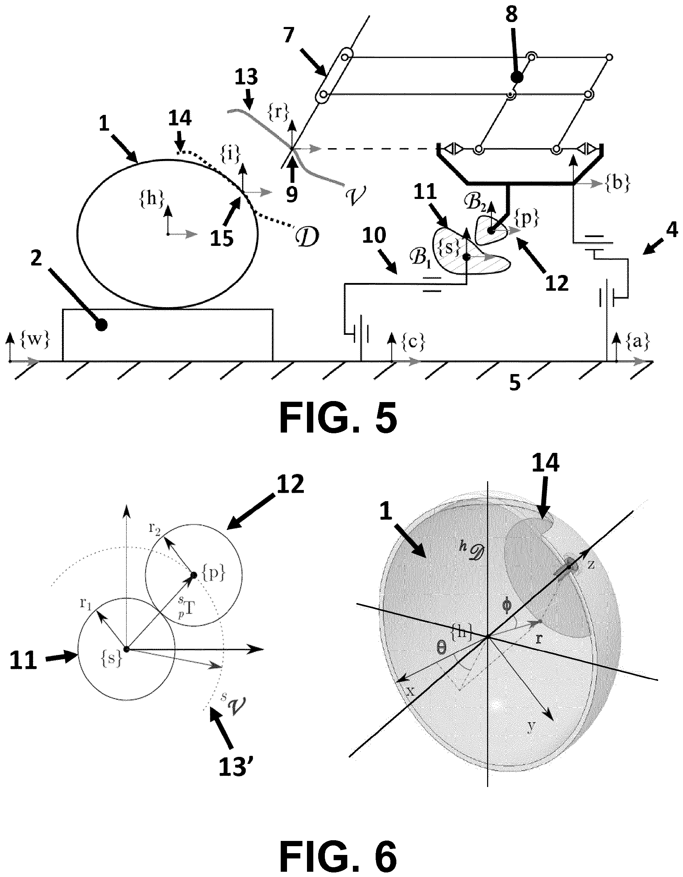

FIG. 5 illustrates a generalized surgical scene using a surgical robotic system architecture with RCM 8 in which embodiments of the present invention can be used. Reference coordinate frames: {a} alignment subsystem base, {b} RCM subsystem base, {r} RCM, {i} surgical entry point, {h} patient, {w} world, {c} fixture mechanism, {s} a first constraining $B_1$, {p} a second constraining $B_2$. The alignment of an RCM mechanism is constrained by a fixture V 13 defined in the world frame. The constraining fixture could be aligned with e.g. a group of admissible RCM locations D 14 based on a patient's anatomy by appropriately positioning the constraining body.

FIG. 6 (left) Side view illustration of the resulting spherical fixture V 13, when choosing $B_1$ 11 and $B_2$ 12 as spheres. (right) An illustration of a spherical region of permissible RCM locations D 14 expressed with respect to the related anatomy h 1 which represents, without loss of generality is, in this illustration, the eye of a patient. Annotated Cartesian and spherical coordinate system conventions are drawn with the origin in eye's center of rotation h, as can be used in embodiments of the present invention.

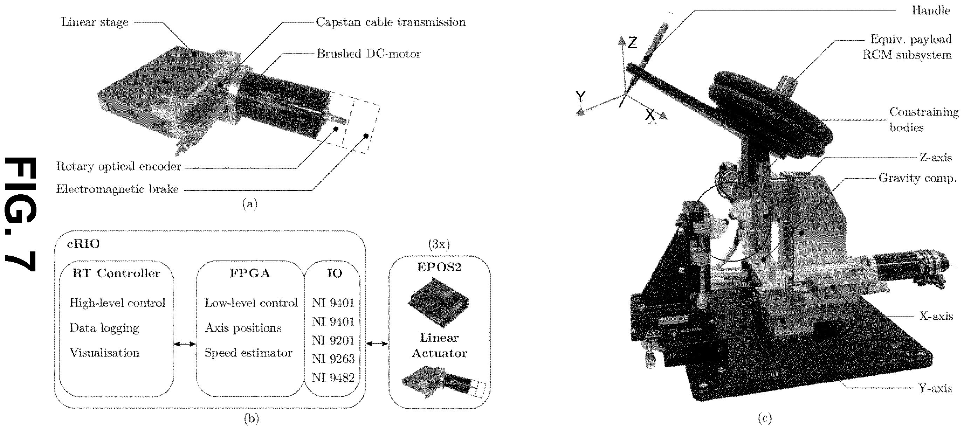

FIG. 7 Overview of the novel alignment subsystem prototype according to one preferred embodiment of the present invention: (a) annotated overview of the custom 1-DoF linear actuator; (b) control architecture overview; (c) annotated overview of the alignment subsystem prototype and mechanical fixture implementation. Three 1-DoF linear actuators were assembled in a gravity-compensated 3-DoF Cartesian configuration.

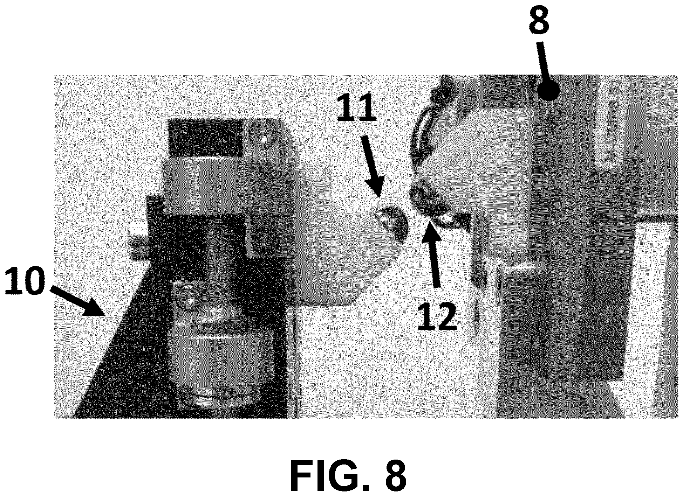

FIG. 8 Close up of a possible fixture implementation for an eye surgery use case, as can be used in embodiments according to the present invention.

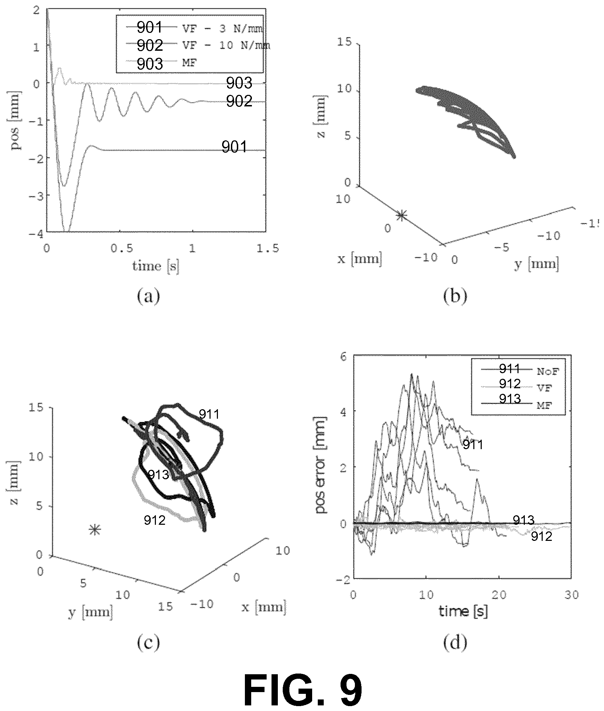

FIG. 9(a) comparison of RCM placement without and with guidance by a virtual fixture and a mechanical fixture. Transient responses when making contact with the target RCM location under virtual damping of virtual damping of 0.16 Ns/mm, illustrating features of embodiments of the present invention; (b) 3D position data validating the ability to constrain the location of an RCM on a complex geometrical shape: validation of the mechanical fixture implementation; (c) comparison of 3D position data during user RCM positioning task without guidance (911), with virtual fixture guidance (912) and mechanical fixture (913); (d) overview of the RCM positioning error as function of time for multiple recordings.

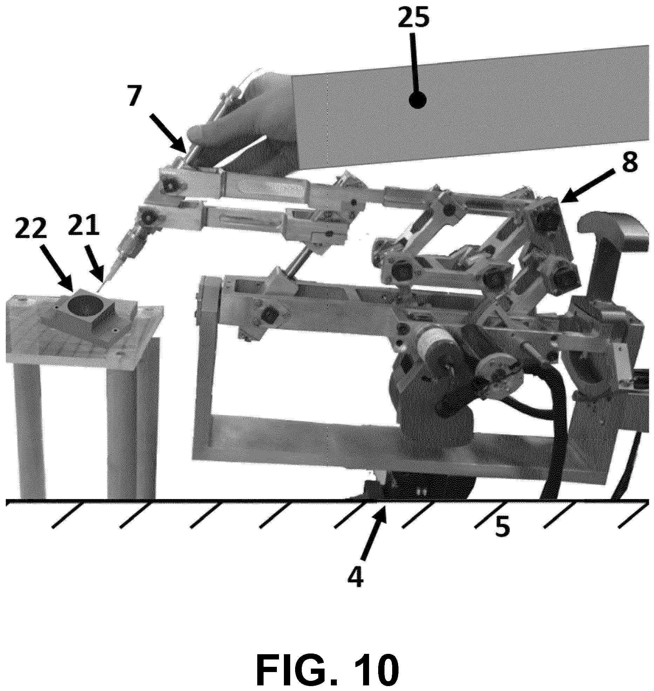

FIG.10 provides an overview of a RCM subsystem 8 jointly manipulating together with a human operator 25 a surgical instrument 7 with an instrument-mounted transducer 21 that is used to measure a certain sample 22 corresponding to the shape of an anatomical structure which can be used to derive patient-specific anatomic boundaries.

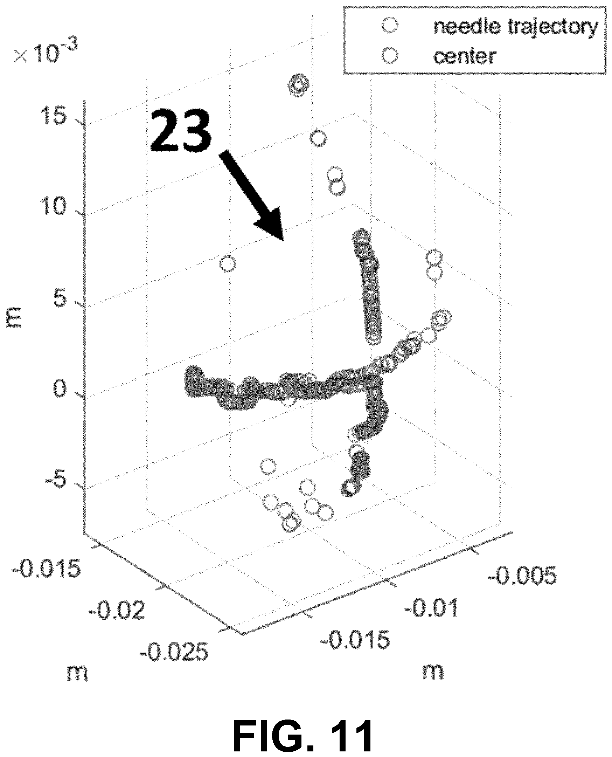

FIG.11 show measurement points 23 of an anatomical structure such as the sample 22 of FIG.10 that can be used to estimate parameters of a model that approximates the anatomical structure which could be e.g. in this case a sphere representing the eye globe whereby the model parameters are the coordinates of the center of the sphere as well as the radius of the sphere.

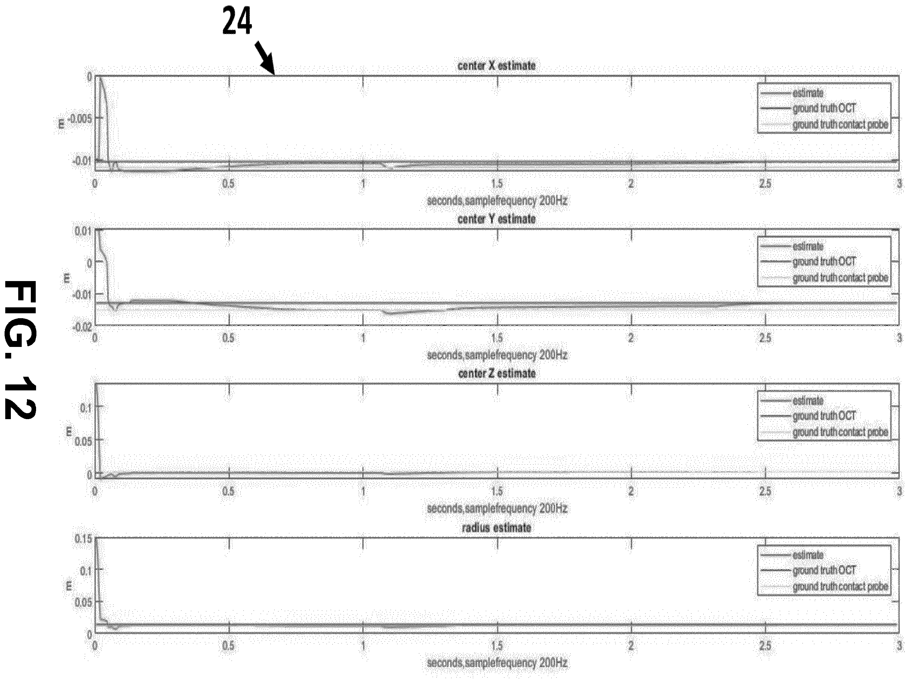

FIG.12 shows the result of an estimation program whereby the parameters of a model such as e.g. the radius and position of the center of a sphere are estimated e.g. with a recursive Bayesian or Kalman filter. Based on the converged estimates a model and associated constraints can then be extracted to be used to guide the operator/system during subsequent manipulations.

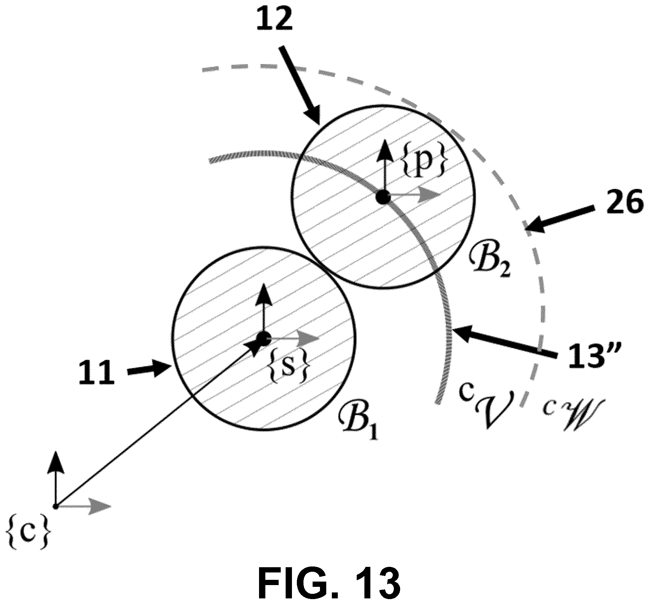

FIG. 13 Composition of a spherical fixture $^cV$ 13" from first constraining body $B_1$ and second constraining body $B_2$, expressed with regards to the fixture mechanism coordinate frame {c}. All constraining points of $B_1$ and $B_2$ are at a given and constant distant from the center of $B_1$ and $B_2$ (coinciding with the origin of frames {p} and {s}).

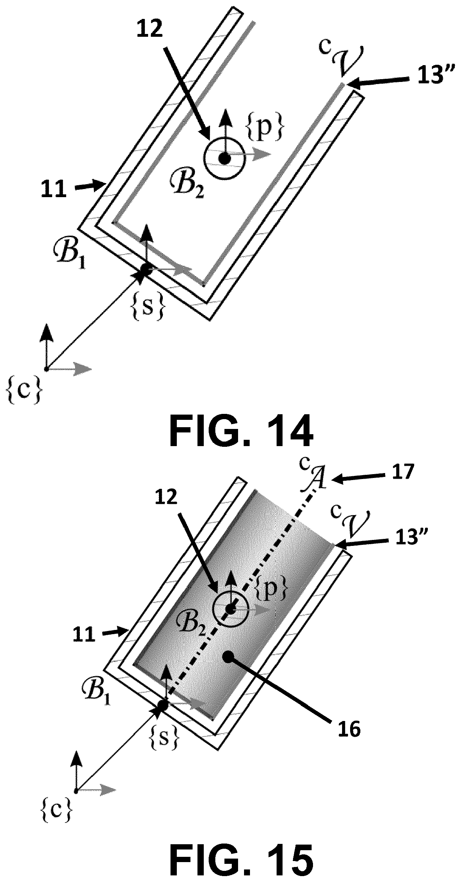

FIG.14 Example of a preferred embodiment combining constraining bodies $B_1$ and $B_2$ to restrain motion to a certain cavity which could be for example used to constrain motion of an RCM point to a region that coincides with the entrance of a natural orifice.

FIG.15 Example of a preferred embodiment combining constraining bodies $B_1$ and $B_2$ to restrain motion to a certain cavity aligned along a certain axis 17 which could be e.g. aligned with a main longitudinal axis of a natural orifice, whereby upon motion through the constraining cavity additional forces are generated, whereby the mechanism for generation of forces could be artificial e.g. by rendering forces via dedicated actuation systems or where the generation of such forces could be physical e.g. by foreseeing some resistive material showing a spring- or damper-like behavior.

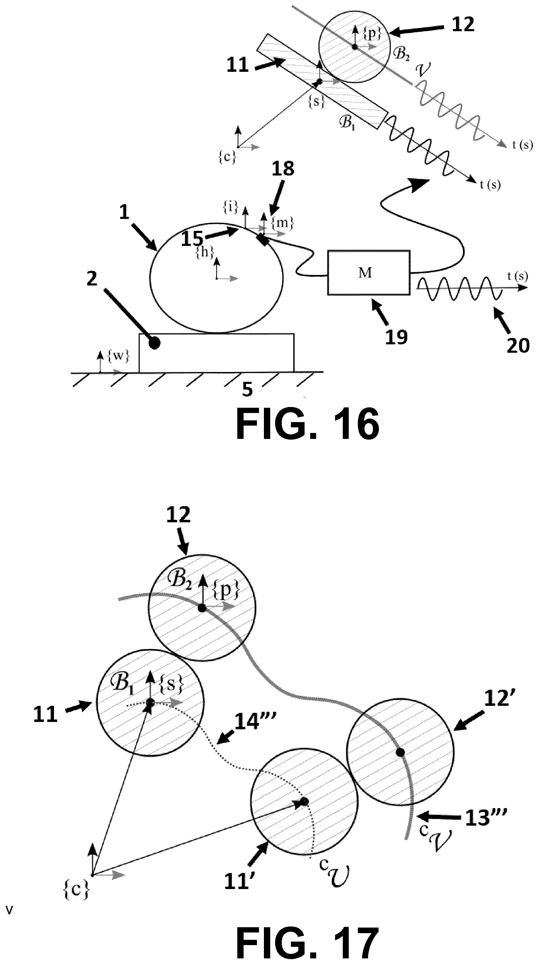

FIG.16 provides an overview sketch of a guidance approach whereby the position of the constraining bodies $B_1$ 11 and $B_2$ 12 are altered dynamically in function of measurement signals detected via a sensor 18 and measurement system 19 at the patient's site.

FIG.17 provides an overview sketch whereby the pose of the constraining body $B_1$ is altered following a certain geometry 14" and in line with variations from the pose of the constraining body $B_2$ such as to create or program a specific shape of boundary surface V 13" which is advantageous for the targeted application.

Table I is summarizing the deviation from the constrained spherical surface while moving the RCM over the said spherical surface. Comparison of RCM positioning task errors without guidance (No Fixture), with guidance from a virtual fixture (Virtual Fixture) and with guidance from the mechanical fixture (Physical Fixture).

## Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

The kinematic architecture of surgical robotic systems can often be generalized as a combination of two subsystems. Without loss of generality, particular examples are given, but the skilled person can easily derive many more configurations with other Degrees of Freedoms (DoFs) and compositions of subsystems that could be arranged to benefit from the here described invention

Where in embodiments of the present invention reference is made to a remote center of motion system, reference is made to a combination of a remote center of motion subsystem and an alignment subsystem, with additionally a fixturing means. The remote center of motion system may in some embodiments be a remote center of motion of a robot.

In a first aspect, the present invention provides a means for assisting in positioning a robot or a mechanism and/or for constraining remote motion of the robot or the mechanism by a mechanical proximal constraining means. Such positioning may be in any suitable environment, e.g. - but not limited to - an operating theatre. The system advantageously is used for providing robot assisted surgery on object, e.g. patient or animals. The object also may be tissue of a human or animal being. Alternatively, the object can also be another type of object. According to embodiments of the present invention, the system comprises means for assisting in positioning of a remote center of motion system, e.g. a remote center of motion of a robot. According to these embodiments, the system comprises a remote center of motion subsystem for establishing a remote center of motion with a number of degrees of freedom to an instrument with respect to an object and an alignment subsystem, coupled to said remote center subsystem, for providing additional degrees of freedom for moving the remote center of motion subsystem with respect to the object of interest. According to embodiments of the present invention, the system furthermore comprises proximal fixturing means. Advantageously according to some embodiments, the fixturing means is based on a hardware mechanical implementation, resulting in increased operational safety. Alternatively according to other embodiments, this may be implemented using controllable degrees of freedom of the remote center of motion system combined with a means of position control of said degrees of freedom. The fixturing means comprises a first body, preferably a rigid body, with known first shape. The first rigid body typically is adjustably fixed to a moveable part of the alignment subsystem. The fixturing means also comprises a second body, preferably a rigid body, with known second shape. The second body typically is adjustably fixed to the environment wherein the remote center of motion mechanism is operating. Further according to embodiments of the present invention, the first body and the second body are shaped and positioned with respect to each other so that contact between the first body and the second body provide restriction of the RCM movement. According to embodiments of the present invention, the first body and the second body are located proximal to the base of the robot. The contact between these proximally located bodies constrains the motion between these proximally located bodies, but results in a restriction of distal motion e.g. on the movement of the distal RCM.

The different features and advantages will be further illustrated with reference to particular exemplary embodiments. It will be clear to the person skilled in the art that the present invention is not limited thereto, but only limited by the claims.

In the example, a general approach for RCM positioning is proposed using anatomy-based haptic fixtures, and is extended with a method and mechanism to implement such fixtures mechanically. After, the proposed approach and method are demonstrated for the use case of robot-assisted eye surgery.

By way of illustration, embodiments of the present invention not being limited thereby, a system according to an exemplary embodiment is described mathematically, illustrating features and advantage of embodiments of the present invention.

Fig.5 depicts a general RCM mechanism 8 mounted on an alignment mechanism 4 that allows positioning of the RCM 9 w.r.t. the surgical entry point 15. Without loss of generality the concept is presented in a 2-dimensional space. It is readily understood that the principle works in 6-dimensional space whereby the 6D pose of the different bodies can be accounted for. The base of the RCM mechanism and the alignment mechanism are marked by the reference coordinate frame {b} and {a} respectively. The RCM mechanism determines a RCM point 9, marked with frame {r} at a known position in space relative to {b}. The adjustment mechanism 4 adjusts the pose of the RCM mechanism's base and subsequently the pose of the RCM point in space. During robot-assisted surgery, one typically controls the adjustment mechanism in a manner such that the RCM point coincides with an entry point 15, marked with frame {i} in the human body 1. Fig.5 shows a frame {h} attached at a specific advantageous location on the human body with respect to which the entry point can be defined. Depending on the surgical application, a multitude of admissible locations of {i} relative to {h} may exist. Depending on the application these locations may be on the surface of an organ, located below the surface of an organ or the skin, inside an natural orifice of the patient, outside of the patient or on any other advantageous location. These locations are represented in Fig.5 by a single region 14 marked as D, but there may be also multiple admissible regions. To simplify aligning the RCM {r}with the admissible set of locations D, a method and mechanism to constrain the adjustment mechanism in space is proposed. Fig.5 shows a fixture mechanism 10 located at base {c} that is adjustable such that a constraining body $B_1$, marked with numeral 11, expressed with respect to {s} can be positioned in space. A counter-body $B_2$, marked with numeral 12, expressed with respect to frame {p} is attached at a fixed location from {r}, for instance at the base of the RCM-mechanism, such that when both bodies contact each other the alignment system is physically prevented in its motion whereby a portion of the workspace of the robot or mechanism is blocked. Consequently, also the positioning of the RCM will be constrained, indicated as a grey line V, marked with numeral 13. By controlling the pose of the fixture mechanism 10, this boundary could be adjusted, shifted or re-oriented conveniently such that its pose coincides with the region D, marked as 14 of desirable RCM locations. It is understood that while the fixture mechanism 10 is depicted with a pair of translational joints in Fig.5 the number and direction of translational joints can be advantageously selected. When the fixture mechanism 10 only contains out of translational joints adjustment of these joints will typically shift the boundary in 3D-space. In case rotational joints are included the boundary could also be reoriented by adjusting one or more rotational joints. In general an adjustment of the entire pose, which consist out of 3 translation degrees of freedom and 3 rotational degrees of freedom, is in principle possible.

By expressing all poses relative to a common world reference frame {w}, that is in a preferred embodiment fixed to the ground (5), the design and alignment can be organised as follows. In general, the location $^w p_r$, of the RCM can be expressed with respect to {w} as:

$$^{w}\mathbf{p}_r = {}^{w}_{a}\mathbf{T}\,{}^{a}_{b}\mathbf{T}\,{}^{b}_{r}\mathbf{T}\,{}^{r}\mathbf{p}_r = f_{wr}(\theta_{\mathbf{a}})$$

$$(1)$$

whereby $^w_a T$ is the fixed transformation matrix from {a} to {w}, also here {a} is in a preferred embodiment fixed to the same ground 5; $^{b}_{r}$T is the, typically but not necessarily, fixed transformation matrix from the rcm {r}to the base of the RCM mechanism {b}; $^{r}$p$_r$ is the location of the RCM with respect to the RCM frame {r}, which is typically simply a zero vector as it coincides with the origin of {r}. Finally, $^{a}_{b}$T is the transformation from {b} to {a}. This matrix is adjustable and a function of the different DoFs, represented by joint vector $\vartheta_a$, of the adjustment mechanism. Whereas Fig.5 depicts a 2DoF alignment mechanism 5 containing 2 translational joints a general system capable of adjusting all the 6 DoFs of the pose can be employed. The above argumentation shows that $^{w}$p$_r$ is fully determined as a function $f_{wr}$ of $\vartheta_a$. If no limitations are provided, the RCM can be positioned using the entire joint space of $\vartheta_a$. In the here proposed scenario, a method is introduced to constrain the RCM such that its movement is compatible with the patient's anatomy. All points $^{s}$p$_{B1}$ of 11, a constraining volume $B_1$, expressed with respect to {s} can be represented relative to {w} as:

$$ ^{w}\mathbf{p}_s = {}^{w}_{c}\mathbf{T}\, {}^{c}_{s}\mathbf{T}\, {}^{s}\mathbf{p}_{B1} = f_{ws}(\theta_{\mathrm{c}}), \qquad (2) $$

whereby $^{w}_{c}$T is the fixed transformation matrix from {c}, the base of the constraining mechanism 10, to {w}; $^{c}_{s}$T is the transformation matrix from {s} to {c} of the adjustable constraining mechanism. As such these are a function $f_{ws}$ of the DoFs $\vartheta_c$ of the fixture mechanism. The point cloud of points $^{s}$p$_{B1}$ of constraining volume $B_1$ is expressed through (2) with respect to {w} dependent on the different values of $\vartheta_c$. Without loss of generality the fixture mechanism is considered to be a non-backdrivable mechanism, such that the pose of this mechanism can be assumed not to be affected by external forces or bodies contacting it. Attached to the base of the RCM mechanism is a second body $B_2$ consisting of points $^{p}$p$_{B2}$ expressed with respect to {p}. Their position relative to {w} is given as:

$$ ^{w}\mathbf{p}_p = {}^{w}_{a}\mathbf{T}\, {}^{a}_{p}\mathbf{T}\, {}^{b}_{p}\mathbf{T}\, {}^{p}\mathbf{p}_{B2} = f_{wp}(\theta_{\mathrm{a}}), \qquad (3) $$

where $^{b}_{p}$T is typically, but not entirely necessary, a fixed transformation matrix from {p} to {b}. Similar to equation (1) the location of the point cloud of points $^{p}$p$_{B2}$ of the constraining volume $B_2$ is adjusted as the joints $\vartheta_a$ are varied. Since both volumes $B_1$ and $B_2$ are considered to be rigid and rigidly connected to respectively the fixture mechanism and the base of the RCM mechanism, these bodies can be assumed not to penetrate each other. In fact, upon contact they will form a rigid fixture that limits the reachable positions that the alignment mechanism can attain. The set of reachable points R for a given $\vartheta_c$ is the collection of joint values $\vartheta_a$ such that $nr_{contact}$, the number of contacts between points $^{w}$p$_{B1}$ of $B_1$ and $^{w}$p$_{B2}$ of $B_2$ is lower than or equal to 1, which is expressed as :

$$ \mathscr{R} = \left\{ \theta_{\mathrm{a}} \middle| nr_{contact} \leq 1 \ , \ \ \forall \theta_{\mathrm{a}} \text{ and } \theta_{c} \right\} \qquad (4) $$

Such can be e.g. computed as the number of cases where the distance d between a point $^{w}$p$_w$ and $^{w}$p$_p$ is smaller than a small positive threshold value $\varepsilon$.

$$ nr_{contact} = \sum \left\{ [c_{pts}] \ , \ \ \forall^{w}\mathbf{p}_{B1} \text{ and } \forall^{w}\mathbf{p}_{B2} \right\} \qquad (5) $$

$$ [c_{pts}] = \begin{cases} 1 & \text{if } d(^{w}\mathbf{p}_{B1}, {}^{w}\mathbf{p}_{B2}) \leq \varepsilon \\ 0 & \text{else} \end{cases} \qquad (6) $$

The values of $\vartheta_a$ where $nr_{contacts}$ is exactly equal to 1 form the boundary of the reachable workspace of the alignment system. Since the pose of the RCM point is typically fixed with respect to the base {b} of the RCM mechanism and due to the fixed transformation from {p} to {b} also fixed with respect to {p}, these same values will determine a remote virtual boundary V through which the rcm $^{r}$p$_r$ cannot pass as depicted in Fig.5. In case where an adjustable RCM is employed, this is a mechanism whereby the mechanism {8} includes means to alter the relative location from the RCM point to the base {b} it is assumed that this alteration is chosen and accounted for appropriately. Now, the shape of the boundary V is determined by the shape of bodies $B_1$ and $B_2$. The pose of the boundary

$$ \mathscr{V} = \left\{ {}^{w}\mathbf{p}_r \middle| nr_{contact} = 1 \ , \ \ \forall \theta_{\mathrm{a}} \text{ and } \theta_{c} \right\} \qquad (7) $$

is determined by the different transformation matrices: $^{b}_{r}$T, $^{b}_{p}$T, $^{a}_{b}$T, $^{w}_{a}$T, $^{c}_{s}$T, $_{s}^{w}$T. Here, all matrices except $^{a}_{b}$T and $^{c}_{s}$T

are typically fixed. The latter, and in particular the related DoFs $\vartheta_c$, can be used to re-locate the pose of boundary $V$ with respect to $\{w\}$. For a given anatomy for which a strategic reference frame $\{h\}$ can be chosen, an expert may be able to derive a preferential portion D of the space - or anatomy - relative to $\{h\}$, e.g. a group of surgical entry point positions i with respect to h such that body wall stress is minimized:

$$^h\mathscr{D} = \{^h\mathbf{p}_i|\text{minimal stress to body wall}\} \tag{8}$$

Once known, $V$ is to be created such that it is isomorphic to D. This can be done for instance by designing $B_1$ and $B_2$ to a specific shape. Once $B_1$ and $B_2$ are known, one can then determine the required transformation $^c_s T$ such that $^w V$ and $^w D$ coincides from observing that in such case $^w p_i$ and $^w p_r$ are to be equal. Therefore

$$^w\mathbf{p}_i = {}^w_h\mathbf{T}\,{}^h_i\mathbf{T}\,{}^i\mathbf{p}_i \tag{9}$$

$$^w\mathbf{p}_r = {}^w_c\mathbf{T}\,{}^c_s\mathbf{T}\,{}^s_p\mathbf{T}\,{}^p_b\mathbf{T}\,{}^b_r\mathbf{T}\,{}^r\mathbf{p}_r \tag{10}$$

so for $^w p_i = {}^w p_r$ and without loss of generality $^i p_i = {}^r p_r = [0001]^T$ equal to each other and to the zero-vector (using homogeneous coordinates), one can rearrange transformation matrices to find $^c_s T$ as:

$$^w_h\mathbf{T}\,{}^h_i\mathbf{T} = {}^w_c\mathbf{T}\,{}^c_s\mathbf{T}\,{}^s_p\mathbf{T}\,{}^p_b\mathbf{T}\,{}^b_r\mathbf{T} \tag{11}$$

$$\left({}^w_c\mathbf{T}\right)^{-1}\,{}^w_h\mathbf{T}\,{}^h_i\mathbf{T} = {}^c_s\mathbf{T}\left({}^s_p\mathbf{T}\,{}^p_b\mathbf{T}\,{}^b_r\mathbf{T}\right) \tag{12}$$

$$^c_s\mathbf{T} = \left({}^w_c\mathbf{T}\right)^{-1}\,{}^w_h\mathbf{T}\,{}^h_i\mathbf{T}\left({}^s_p\mathbf{T}\,{}^p_b\mathbf{T}\,{}^b_r\mathbf{T}\right)^{-1} \tag{13}$$

$$^c_s\mathbf{T} = {}^c_w\mathbf{T}\,{}^w_h\mathbf{T}\,{}^h_i\mathbf{T}\,{}^r_b\mathbf{T}\,{}^b_p\mathbf{T}\,{}^p_s\mathbf{T}, \tag{14}$$

whereby $_s{}^p T$ corresponds to the transformation matrix from $\{p\}$ to $\{s\}$ when both bodies are contacting each other. Note that $^h_i T = {}^h_r T$ in this case. Also note that all transformation matrices in (14) are known apart from $^w_h T$. Therefore a registration procedure must be done prior to the intervention to identify $^w_h T$. Once $^w_h T$ is determined the required values for $\vartheta_c$ may be directly computed from (14), and used to drive the fixture mechanism such that $^w V = {}^w D$. Once positioned, one should fix $\vartheta_c$, at which point one can safely re-position the RCM through varying the joint positions $\vartheta_a$. To demonstrate proof of concept, this is applied for the use case of vitreoretinal surgery.

Further by way of illustration, the described method is applied for the use case of eye surgery. During eye surgery surgical ports are placed through the sclera, an approx. 1 mm thick tissue, at 2.5 to 4 mm from the corneal limbus edge. Permissible RCM locations are considered to lie inside the surgical port at the center of the scleral wall, rotated about the eye's natural center of rotation. As such, the surgical port and RCM remain aligned when rotating the eye. Mean human anterior radius and corneal limbus diameter are 12.1 mm and 11.8 mm respectively [12]. For the purpose of this description, a spherical eye with anterior radius and corneal limbus diameter 13.2 mm and 12.9 mm respectively - being 9 percent larger than mean - is assumed as patient anatomy. A single surgical port at 4 mm from the corneal limbus at 3 o'clock is taken as example of an anatomical reference point. This would result in a spherical fixture with a radius of 12.7 mm (Fig. 6). As a first indication for angular range, the required angle to rotate the edge of the corneal limbus to the center of the microscopes optical path is considered. At this point, meaningful visualisation is lost. This is estimated to be 30° in any given direction from the surgical entry point and about the eye's center. Given these considerations, the region of permissible RCM locations D can be expressed as a range of spherical coordinates $r = 12.7mm, \varphi = 0...30°, \vartheta = 0...360°$ (Fig. 4).

In order to achieve this, $B_1$ and $B_2$ are both designed as spheres with radii 6.35 mm (Fig. 4). By utilizing the point-contact between the two spheres with known radii $r_1$ and $r_2$, a spherical fixture V with radius ($r_1 + r_2$) is created which has its origin at the center of $B_1$. The initial pose between both bodies is set based on the known location of the surgical entry point {i} w.r.t. the eye's center {h}.

In yet a further illustration, a particular example of a surgical robot RCM positioning is illustrated using anatomy-based haptic fixtures. A prototype of an alignment subsystem and fixturing means based the abovedescribed embodiment is detailed in Fig. 7 and Fig 8.

The illustrated prototype consists of three custom 1-DoF linear actuators assembled in a Cartesian configuration, offering a 50x50x50 mm workspace (Fig. 7c). The RCM subsystem mass is simulated by a static load of aprox. 6 kg, which is applied using commercial barbell weights. The vertical stage of the example shown is equipped with a spring-actuated assembly providing primary gravity compensation. The residual gravitational force, aprox. 2 % force variation across the full Z-axis workrange, is identified and actively compensated with a 3rd order polynomial fit.

Custom 1-DoF linear actuators with high backdrivability were developed (Fig. 7a). Each contains a commercially available linear stage (M-UMR8.51, Newport Corporation, USA), providing a 50 mm workrange at 900N centralized equivalent static load capacity. The linear stage is paired with a precious metal brushed DC-motor (448590 Maxon Motors, Switzerland) and a 5000 CPT rotary optical encoder via a custom aluminum assembly. An electromagnetic brake is added in order to rapidly immobilize the stage when needed. A dual capstan cable transmission with a pulley radius of 5 mm and 0.6 mm diameter 6x19 AISI 316 cable rope is used to transmit torque to the linear stage, providing 17.6N max force output at 1,7 $\mu$m positional resolution.

Low level and high level control are in the present example done using motor drivers (Epos2 50/5, Maxon Motors, Switzerland) paired with FPGA and a RT embedded controller (cRIO 9035, National Instruments, USA) respectively (Fig. 7b). Motor drivers are used in torque control mode, while position and speed estimation are done on FPGA. High level control is done in real-time using LabVIEW 2017 (National Instruments, USA). Parallel real-time loops are implemented for high-level control, data-acquisition, and visualisation at 1 kHz, 200 Hz, and 30 Hz respectively.

As a first prototype for the fixture mechanism, three manual linear stages (M433, Newport) in combination with lockable manual precision screws were assembled in a Cartesian configuration (Fig. 7c). These formed the fixture mechanism, and were mounted to the base plate of the alignment subsystem prototype. Two hardened steel precision-balls with radii of 6.35 mm were used to create the required fixture shape while offering minimal friction during contact. These were glued into custom 3D-printed holders, and were fixated opposite to each other on the fixture mechanism and alignment subsystem. The resulting fixture shape is determined by the point contact condition between the spheres, and the mechanical limits of the 3Dprinted holders. Each sphere is inserted halfway into its holder at a 45 °angle from its mounting plane, placing angular mechanical limits well beyond the earlier identified requirements. Prototype stiffness is approximated to be 1.4 kN/mm using combined values from Hertzian contact theory for the spheres and FEA-modelling for the 3D-printed holders. Note that this specification does not include the overall system stiffness, and is thus not representative for fixture stiffness.

By way of illustration,
an initial experimental validation is performed in order to review feasibility. First, the implementation limit for virtual fixtures is experimentally determined. Second, a comparison is made between the virtual and physical fixture implementations in terms of penetration error and transient response. Third, the mechanical fixture shape is verified. Fourth, an initial review of user positioning errors during a simulated surgical case is made for a mechanical, virtual, and no fixture case.

The achievable virtual stiffness was experimentally determined for a heuristically chosen virtual damping level of 0.16 Ns/mm. A 1 DoF virtual FRF, also known as a virtual wall, with a stiffness of 1N/mm is implemented at a known location in the z-axis workrange. With the other axes immobilized, a 0.5 kg mass is added to the z-axis which is left uncompensated. When released from the upper workspace limit towards the virtual fixture, this causes a repeatable worst case fixture interaction, while producing a constant steady-state force of approx. 5 N after transient. This process is repeated for stiffness increments of 1 N/mm until a transient oscillation is observed which does not decay within 1 s, which is considered the implementation limit. Computed forces for rendered fixtures are limited in software to $\pm 10$ N. The implementation limit is determined to be 10 N/mm.

The above described experiment was repeated for the mechanical fixture. A relative comparison between mechanical fixture and selected virtual fixture equivalents can now be made. Fig. 9a highlights selected experimental results. The transient responses of the mechanical fixture (MF), the stiffest virtual implementation (VF - 10 N/mm), and a virtual implementation with comparable settling time to the MF response (VF - 3 N/mm) is shown. As one would expect, a clear difference is visible when comparing steady state values between VF's and MF due to a higher stiffness. Due to lack of measurement resolution and load size, no meaningful stiffness estimation can be made at this time. A difference in transient response is visible. The mechanical fixture achieves a faster steady state value when compared to its stiffest virtual counterpart (VF - 10 N/mm). A trade-off between settling time and steady state error can be made by lowering virtual stiffness, as is shown by (VF - 3 N/mm). Furthermore, while the virtual fixtures dissipate the impact through large overshoot, the mechanical fixture exhibits an irregular, high frequency transient. It is hypothesized that due to the high stiffness contact, more impact energy is preserved, resulting in a brief bouncing behaviour of the contact bodies. During the experiments, a minor excitation of the alignment subsystem is observed. It is assumed that a much larger portion of the preserved impact energy is dissipated through the subsystem assembly when using high-stiffness mechanical fixtures. This should be taken into account during design and mitigated if needed, as this might be a stability concern for closed-loop impedance control.

The implementation of the earlier described mechanical spherical fixture is validated on shape. A user is asked to randomly track the fixture shape at a comfortable force level for approx. 60 s. After, a spherical center is numerically fitted, and used to compute a radial error by taking the difference between the desired radius of 12.7 mm and the Euclidian distance between each measured point and the fitted sphere's center. Fig. 9b provides a 3D visualisation of the recorded positions. It is clear that the fixture implementations indeed creates a spherical fixture. Mean radius was found to be 12.66 mm with a $\pm 2\sigma$ range of 0.024 mm.

As a first use case validation, RCM positioning accuracy is reviewed. This is defined as the user's ability to position the alignment subsystem in such a way that the RCM coincides with a previously identified set of desired locations. For the purpose of this experiment, a simple rigid mock-up of the eye was developed based on the earlier described eye dimensions. A gimbal mechanism is used to allow for 2 rotations about the eye's center. 7 visual landmarks were added to the posterior segment of the eye; One in the center of the field of view, and 6 which require rotation of the eye in order to visualize. The surgical instrument is fixed at a known location and pose to the alignment subsystem. This resembles an RCM subsystem of which all DoF are locked. The currently fictitious RCM is chosen to be a fixed point on the instrument shaft, providing 15 mm of instrument length inserted into the eye when aligned with the surgical entry point. At the start of each experiment, the positions of the eye model, alignment subsystem, and - if applicable - fixture mechanism are initialized to the same pose. The user is asked to reposition the surgical port using the alignment mechanism, thus rotating the eye, in order to visualize all landmarks. This is done in a clockwise sequence, starting and ending by centering the central landmark. This experiment is conducted 6 times for three cases: 1) no guidance fixture, 2) virtual guidance fixture, 3) mechanical guidance fixture. All positioning tasks were done with 0.16 Ns/mm virtual damping. Virtual fixture stiffness was set to 10 N/mm. In order to resemble eye-surgical operating conditions, no visualisation of the RCM or the surgical entry point are provided. Since the mock-up does not include eye optics, no corrective optics or surgical microscope are used. The user views the surgical scene via a monitor, captured using a USB camera fixated above the eye. As a first validation, a single expert user conducted the experiments. A total of 18 datasets were collected (3 cases, 6 experiments each) using the alignment subsystem encoders. The positional error is computed as the Euclidean distance between the measured RCM position and the nearest acceptable RCM location based on the fixture shape. Fig. 9c shows 3D positional data of a single experiment for each case. A similar tracking pattern is visible for both fixture cases, while a clear positional drift from the tracked surface is visible without the usage of a guidance fixture. This is further highlighted in Fig. 9d, which shows the positional RCM placement error in function of time for all recorded cases. Here, a systematic difference between the three cases is clearly visible. This is further detailed in Table I, showing median, minimum, and maximum positional errors. Positional error values systematically decrease approx. one order of magnitude between the no fixture and virtual cases, as well as between the virtual and mechanical cases.

TABLE I: User RCM positioning error

| Experiment | Median [mm] | Min [mm] | Max [mm] |
|---|---|---|---|
| No Fixture | 1.40 | -1.17 | 5.34 |
| Virtual Fixture | -0.13 | -0.47 | 0.37 |
| Physical Fixture | -0.04 | -0.06 | 0.03 |

The above experimental data illustrate the feasibility of method embodiments according to the present invention. Both a virtual and mechanical implementation of the proposed RCM fixtures were successful at aiding a user during a surgical visualisation task requiring RCM changes. Furthermore, the mechanical fixture outperformed the virtual and no fixture cases in terms of positional error, with one and two orders of magnitude respectively. As such, a higher-stiffness alternative to fully virtual implementations are provided according to embodiments of the present invention. While the presented guidance method and mechanical fixture method can be generally applied during robot assisted surgery, the latter's advantage is believed to be most prominent when using robotic systems which are limited in rendering virtual stiffness or are required to accurately and safely constrain motion of larger payloads. Additionally, passive or under actuated systems might also find value in the presented mechanical fixture approach. The experimental validation identified a key design consideration for mechanical fixtures, being dissipation of the initial contact impulse. While minor for this use case, neglecting this could lead to undesired high-frequency excitation of the robotic system, thus creating a stability concern. Should mitigation be required, in particular embodiments additional damping could be implemented, contact between both constraining bodies could be ensured, and/or a tuned mass damper could be added to the fixture mechanism. Currently, a spherical assumption for eye anatomy is used. While this in some embodiments is sufficient for close-to-mean anatomy, in some embodiments a more accurate model is used for higher patient inclusivity. In order to accurately construct such a surface, embodiments can for example take into account knowledge of the eye's anterior radius, limbus diameter, and center of rotation based on the level of myopia/hyperopia. Patients undergoing vitreoretinal surgery already receive preoperative imaging capable of collecting this data such as OCT (Optical Coherence Tomography) imaging.

In some embodiments generation of the proposed RCM fixture geometry could rely on intraoperative imaging whereby the geometry of the anatomical feature is estimated on the spot based on any relevant sensor which could be sensors that make contact with the anatomy such as but not limited to force, pressure, tactile sensors or by non-contact sensors based on radioactive, optical, ultrasound or other waveforms that allow imaging or reconstructing the patient anatomy.

Without loss of generality an example is provided for vitreoretinal eye surgery where for example intra-operative Optical Coherence Tomography is used to estimate the radius and the location of a sphere model representing the eye globe. In one preferred embodiment an optical fiber is integrated into a surgical instrument and connected to an Optical Coherence Tomography system offering an instrument with integrated intra-operative imaging capability. Fig.10 shows a surgical instrument 7 namely a needle with an iOCT fiber that is integrated along the longitudinal axis of this needle. The fiber forms an instrument-mounted transducer 21 that provides, after processing the raw iOCT signals, a signature of the material that is located along a straight line ahead of the optical fiber. By applying filter or thresholding techniques to this measurement, the said instrument can be used to estimate the closest distance $dt_k$ to tissue in front of the optical fiber. For each pose k of the instrument such measurement will be obtained. Fig.10 shows how this instrument is mounted in an RCM robot 8 and e.g. be jointly held by the said robot 8 and the hand/arm of a human operator 25. Where the position of the instrument held by the robot can be computed with respect to a world frame {w} by knowing the full robot pose, the position of the anatomical tissue that is seen by the iOCT fiber can be expressed also with respect to {w} e.g. by computing the position of the tip of the instrument held by the robot and computing the point at a distance $dt_k$ ahead of the tip along the direction of the instrument's longitudinal axis. In the case the human body can be assumed to be stationary and sensor noise can be neglected or is averaged out, each instrument pose will thus generate a measurement point of the human anatomy that can be expressed with respect to the world frame. In an alternative embodiment the instrument could also be fitted with optical markers, electromagnetic sensors or any kind of sensor that can track the instrument and express its pose with respect to the world frame {w}. Equipped with such measurement tool a large number of measurements could be generated of the human anatomy or any targeted object. Fig.11 shows for example a set of measurement points 23, forming a so-called point cloud of data, that were obtained by moving the mentioned instrument with iOCT sensing capability over a hemispheric object, with the same dimensions as the inside of a human eye, which is referred as numeral 22 in Fig.10. Whereas the individual data points may be noisy, by employing filters such as e.g. a Kalman filter, the noisy data can be used to estimate the parameters of a model of the anatomy. Fig.12 shows for example how the x- (in the upper row), the y- (in the 2nd row), the z- coordinate (in the 3rd row) of the center of the sphere as well as the radius (in the bottom row) can be estimated based on this noisy data. The Figure shows how already after 0.1 seconds, based on 200Hz iOCT measurement rate, fairly good estimates of these parameters are obtained. This approach can be useful to estimate the center and radius of the eye globe which can be used to derive the shape of a target constraining surface $^w D$. Other approaches to estimate and localize the eye globe may be adopted as well.

Whereas the proposed estimated method may be used to constrain the locations of an RCM of a RCM mechanism to a certain region in space delimited by one or more boundaries $^w D$ the method is not limited to constrain RCM mechanisms. In fact, the proposed method can be adopted more generally to constrain remote motion of a robot or mechanism through proximally located mechanical constraining means. Fig. 2 shows such a general robot or mechanism 3 that is mounted on an alignment stage 4 holding an instrument 7 at its end-effector 6 which may be used to treat a patient 1 lying on an operating table 2. Where the alignment system 4 can help adjust the workspace of the robot or mechanism 3 conveniently with respect to the patient to allow reaching targeted regions it may be advantageous to restrict the motion to certain safe zones. Along similar lines and making use of a fixture mechanism 10 and constraining bodies $B_1$ and $B_2$, one can constrain the movement of the surgical instrument 7 to certain regions V expressed in relation to robot and/or alignment system and regions D relative to the object or the patient to be operated upon. For example based on the identified hemisphere in Fig.11 and Fig.12 one could compute a region D in such a way that the instrument would never be allowed to approach the retina (or any other anatomical feature) closer than a certain distance. Alternatively one could allow a penetration relative to D provided it is not deeper than a certain threshold value.

Whereas the previous description discussed how to constrain remote motion by installing a boundary V at an appropriate location it is a further aspect of this invention to allow installing multiple of such boundaries e.g. by foreseeing additional sets of $B_1$ and $B_2$ pairs that constrain a different portion of the remote space. It is a further object of this invention to foresee a means to quickly lift such boundaries. E.g. one can easily imagine a clutching device in the vicinity of either of bodies $B_1$ and $B_2$ to move either or both of these bodies out of the way or oppositely move it back in place. For example in relation with Fig.13 one could image to install a remote constraining region at $^c V$ such that the instrument or RCM can be positioned against that boundary and that, after to instant where contact with the boundary has been established, a second boundary $^cW$ would be brought in place such that the instrument or RCM is constrained in a bilateral fashion and force to move between $^cV$ and $^cW$ if the distance between $^cV$ and $^cW$ is close to 0 the instrument or the RCM would be constrained fully only capable of moving along the surface of $^cV$. For example in robotic eye-surgery when moving an RCM mechanism the RCM could be made to move on a sphere such that the RCM will always be located in central on the sclera to minimize stress on the tissue. In contrast to the unilateral constraint where the RCM could drift outwards leading to excessive forces on the scleral tissue this bilateral constraint could simplify and enhance safe operation. Whereas the second constraint $^cW$ could be created by a 2nd pair of mechanical constraining bodies $B_1$ and $B_2$ which could be connected at different locations to the robotic subsystem 3, RCM subsystem 8, alignment subsystem 4 or fixture mechanism 10 compared to the first pair of constraining bodies $B_1$ and $B_2$, in alternative embodiments this second constraint could be also created through software as virtual constraint which could be easily switched on or off. In such case the bilateral constraint would have one side that is realised by mechanical hardware and a second side that is implemented in software. A limitation of the proposed mechanical method is its reliance on the physical interaction between predefined bodies to represent the required fixture. Therefore, it is inherently less flexible when compared to fully virtual fixtures with regards to matching a generic fixture shape. However, in the case of RCM positioning, it seems reasonable to assume that the required level of detail might be limited, and that a specific combination of bodies might accommodate for a range of procedures and patient anatomies. For instance, a sphere in a hollow cylinder could be used during natural orifice surgery, during which the fixture bodies and thus the predefined RCM workrange - are shaped based on a percentile range of human anatomy. As such, a single setup and fixture body combination could be used for a broad range of patients and procedures, with a reasonably chosen anatomical error.

FIG.14 shows for example how in some embodiments the proposed invention could be used in natural orifice surgery to allow free repositioning of an instrument or of an RCM point within the natural orifice, but where the proposed method installs a constraint $^cV$ to prevent departing from the orifice potentially damaging the tissue that makes part of the orifice's lumen. The shape $^cV$ could be a detailed patient-specific shape or it could be a generic shape that is a simplistic subset of the orifice or an overestimation that still limits excessive departure from the orifice.

Where in a preferred embodiment of the invention the alignment subsystems are back-drivable allowing unhindered motion when not constrained, in alternative embodiments a certain form of resistance or guiding force under the form of spring, damping or inertial forces may be foreseen depending on the targeted application. E.g. FIG.15 shows a force field 16 marked as greyscale region whereby the intensity of the figure is a representation of the intensity of the resistive force that is generated to prevent motion or of the guiding force that is generated to preferentially align towards a preferential region relative to the force field. The force field depicted in FIG. 15 preferably aligns the body $B_2$ to a preferential region within a constrained volume and by doing so it thus also preferably aligns the remote instrument or a RCM point to a corresponding remote preferential region. In the Figure this preferred region is arranged nearby a central axis $^cA$ 17 but any other valuable portion may be foreseen. In the preferred embodiment depicted on FIG.15 the force field simplifies motion through, along or towards this preferred region and prevents, but not fully limits, movement in the regions surrounding this preferred region. Whereby this preferred region stretches in the preferred embodiment depicted in FIG.15 up to the constraints 13" marked as $^cV$. It is understood that such behaviour could be done through software e.g. by rendering virtual stiffness, damping, mass or other behaviour. It is understood that such behaviour may also be realized through implementing it relying on physical phenomena e.g. by attaching a spring or introducing oil-like substance or similar.

Where the constrained region can be preferentially implemented to improve safety in the vicinity of fragile structures of an object or the patient's body or to ease or enhance the efficiency of working in the vicinity of such structures, it is a further object of the presented invention to allow adjusting the constraint regions in a dynamic fashion such that if the remote fragile structures alter dynamically the constraint regions can be altered dynamically as well such that relative motion between the targeted region and the instrument or RCM can be reduced. Whereas different information sources may be employed to capture the dynamic behaviour of the structures one wishes to work upon FIG.16 shows a preferred embodiment where a motion tracking sensor 18 with reference frame {m} is positioned in the vicinity of the area of interest, such as for example 15. A measurement system M 19 that is wired, but may also be wireless connected to the tracking sensor 18, that captures the motion of said tracking sensor, can be used to generate a signal 20 in function of time, which on its turn may be used to alter the motion of the proximal constraining bodies $B_1$ and $B_2$ and by doing so altering the motion of the constraining surface V and the corresponding remote constraint or constraints. It is an advantage of some embodiments of the invention that the location of $B_1$ and $B_2$ can be updated such that the remote constraining surface updates to maximally align with the motion of the site of interest 15 whose motion is estimated through measurement of the motion sensor 18. Whereas FIG.16 shows a tracking sensor that is mounted on the structure of interest, the estimation of the motion could also be estimated by an array of motion sensors which may be mounted. In other embodiments the sensor or array of sensors are not in direct contact but may be within line of sight. It understood that any type of sensor that may be used to estimate the motion, through measuring the pose, the velocity or acceleration can be employed advantageously in this setting. Alternatively the sensor could also measure force, stress, strain, or deformation to adjust the constraint dynamically based on these sensor signals and e.g. limit the possible interactions to safe limits.

Furthermore, a conceptual expansion of the physical fixture approach is envisioned, allowing for programmable geometries to be rendered by active positioning of the fixture mechanism as conceptually sketched in FIG.17. The figure shows how a complex constraint region $^cV$ spanning from location 12 to location 12' along a complex path or surface 13" is achieved by altering the position of body $B_1$ in relation to $B_2$ from location 11 to 11' along a complex corresponding path or surface 14'''. This would increase the flexibility of the approach, as it would allow for using a single combination of bodies or a limited combination of bodies to represent a multitude of fixture shapes. As such, a single dedicated setup or limited number of dedicated setups could be used for a range of patient anatomies and procedures. For instance, during eye surgery a single set of spheres in combination with a position controlled fixture mechanism could be used to represent a range of spherical and ellipsoidal fixtures. In such case, fixture rendering would be limited by the bandwidth, precision and stiffness or control stiffness of the controlled fixture mechanism and other subsystems. When using this approach e.g. to actively render accurate geometries that help constrain an RCM, it is assumed that the positional rate of change of the robot's RCM should be limited to a fraction of the control rate of the fixture mechanism. As currently implemented, the resulting mechanical fixture acts as a FRF. At this point, surface tracking requires the user to exert force towards the patient. In the case of eye surgery this might be a limiting factor, as repositioning might be desired while retracting the instrument; GF behaviour is preferable. This could be resolved in some embodiments in a similar way as depicted in FIG.13 by adding a virtual attractive force field on the outside of the mechanical fixture geometry, resulting in a hybrid between mechanical and virtual fixtures or by complementing the system with multiple programmable bodies $B_1$ and $B_2$ that are controlled appropriately.

In some embodiments, the fixture mechanism is based on bodies $B_1$ and $B_2$ that result in a cylindrically shaped fixture. Such can be achieved with a sphere placed inside a cylindrical cavity. Such a fixture would be particularly advantageous during NOS, where RCM placement is ideally restricted to a cylindrical point cloud of permissible RCM positions.

In some embodiments, the fixture mechanism is based on bodies $B_1$ and $B_2$ that result in a plane shaped fixture. Such can be achieved with a sphere placed on a plate. Such a fixture would be particularly advantageous during MIS through a body wall, where the body wall could be locally represented by a planar point cloud of permissible RCM positions. Other shapes and forms can be engineered. In yet other embodiments, the fixture mechanism is based on a body $B_1$ which is actively positioned w.r.t. $B_2$ in order to produce a fixture shape in the robot coordinate frame {a} which differs from the resulting fixture shape in the robot coordinate frame {a} when $B_1$ would be kept stationary. Such an embodiment would be particularly advantageous as it would allow for creating a variety of fixture shapes with a single set of bodies. For instance, two spherical bodies could be used to produce an ellipsoidal fixture shape. This embodiment could rely on a control algorithm which produces a desired location for $B_1$ w.r.t. $B_2$, based on the positions of a desired fixture shape V and the known shapes of $B_1$ and $B_2$. It is an advantage of at least some embodiments of the presented invention to implement the fixture shape V through any combination of bodies $B_1$ and $B_2$ whereby the number of bodies does not need to be equal. For example a plurality of bodies $B_2$ could be used to constrain a single body $B_1$.

Reference numbers

**[0118]**

1. Patient
2. Surgical table
3. Robotic subsystem
4. Alignment subsystem
5. Stable base-floor
6. Robotic End-effector
7. Surgical instrument
8. RCM subsystem
9. Remote Center of Motion (RCM)
10. Adjustable fixture mechanism
11. Constraining Body B1
12. Constraining Probe Body B2
13. Robot-centered Constraint Motion Boundary Surface V
14. Patient-centered Constraint Motion Boundary Surface B
15. Patient incision
16. Force field
17. Anatomic reference axis
18. Body-mounted transducer

19. Intra-operative measurement system
20. Dynamic motion signal
21. Instrument-mounted transducer
22. Test sample
23. Measured test sample
24. Estimated model parameters
25. Human operator
26. Multiple boundary

**Claims**

1. A system for assisting in constraining remote motion of an instrument and/or of a remote portion of the system in a working environment, the system comprising

   - a robotic subsystem or mechanism for positioning the instrument and/or the remote portion of the system for assisting in positioning with a number of degrees of freedom with respect to an object,
   - an alignment subsystem, coupled to said robotic subsystem or mechanism, for providing additional degrees of freedom for moving the instrument and/or the remote portion of the system with respect to the object of interest,

   the system for assisting further comprising a fixturing means, the fixturing means comprising
   a single first body with known first shape or a plurality of first bodies with known first shapes, referred to as the probe, the probe being adjustably fixed to a moveable part of the alignment subsystem, and
   a single second body with known second shape or a plurality of second bodies with known second shapes, referred to as the probed surface, the probed surface being adjustably fixed to an operating theatre wherein the robotic subsystem or mechanism is operating, whereby the probe and the probed surface are shaped and positioned with respect to each other so that contact between the probe and the probed surface provides restriction of the instrument movement and/or of the movement of the remote portion of the system.

2. A system according to claim 1, whereby the robotic subsystem or mechanism contains or is a remote center of motion (RCM) mechanism, whereby the system assists in constraining the movement of the remote center of motion point and where the contact between the probe and the probed surface provides this restriction in movement of the remote center of motion mechanism and the remote center of motion point.

3. A system according to any of claims 1 or 2,
   wherein the shape of the probe and/or the shape of the probed surface is determined as function of the anatomy of the object, and/or
   wherein the probe and/or probed surface are rigid bodies.

4. A system according to any of claims 1 to 3, wherein the position of the probe and/or the position of the probed surface is determined as function of the anatomy of the object.

5. A system according to any of the previous claims, wherein the fixturing means is configured for keeping the probe and the probed surface into contact with each other for defining a mechanical restriction for the instrument movement and/or the movement of a remote portion of the mechanism and/or the movement of a remote center of motion point.

6. A system according to any of the previous claims, wherein the alignment subsystem is rigidly coupled to the robotic subsystem or mechanism and/or wherein the alignment subsystem is rigidly coupled to the remote center subsystem.

7. A system according to any of the previous claims, wherein the probed surface is fixed to the working environment via a further alignment subsystem, allowing controlled positioning of the probed surface with respect to the probe.

8. A system according to claim 7, wherein the further alignment subsystem provides the same degrees of freedom as the alignment subsystem to which the probe is adjustably fixed.

9. A system according to any of the previous claims, wherein the probe and the probed surface, when into contact, form any of a sphere on a sphere, a sphere on a plate or surface, a sphere in a hollow cylinder or a cylinder on a plate or surface.

**10.** A system according to any of the previous claims, wherein the fixturing means is configured for keeping the probe and the probed surface into contact with each other for defining a mechanical restriction for the instrument motion or for the movement of an RCM.

**11.** A system according to any of the previous claims, wherein the system furthermore is configured for providing a virtual force field that attracts, repels, slows down or stabilizes the motion of the instrument or the movement of a remote portion of the mechanism or of the motion of the remote center of motion.

**12.** A system according to the previous claim, wherein the system is configured for providing a virtual attractive force field for repositioning the instrument or a remote center of motion along a surface defined by different positions of the probe and the probed surface when the probe and the probed surface are in contact with each other.

**13.** A system according to any of the previous claims,
wherein the system is configured for allowing instrument repositioning or repositioning of a remote portion of the mechanism or repositioning of a remote center of motion point during a surgical intervention, and/or
wherein the system is configured to estimate the state or the alteration of the state of a remote object and whereby the position of the probe and / or the position of the probed surface is altered in response to measured state or the alteration of the state of the remote object, and/or
whereby the position of the probe and the probed surface are altered in a coordinated fashion such as to advantageously program the shape of the region where instrument movement or movement of a remote portion of a mechanism or of a remote center of motion point is restricted.

**14.** A robot for use in an operating theatre, the robot comprising a system for assisting in positioning of a remote instrument and/or of a remote portion of the system or of a remote center of motion point of the robot according to any of the previous claims.

**15.** A method for assisting in positioning of a remote center of motion of a robot, according to claim 14, in an operating theatre, the method comprising positioning a remote center of motion with respect to an object by controlling positioning of the remote center of motion along an anatomy-derived fixture shape.

**FIG. 1**

**FIG. 2**

FIG. 3

FIG. 4

FIG. 5

FIG. 6

Handle
Equiv. payload
RCM subsystem
Constraining bodies
Z-axis
Gravity comp.
X-axis
Y-axis

(c)

(3x)
EPOS2
Linear Actuator

Capstan cable transmission
Brushed DC-motor
Linear stage
Rotary optical encoder
Electromagnetic brake

(a)

cRIO

| RT Controller | FPGA | IO |
|---|---|---|
| High-level control | Low-level control | NI 9401 |
| Data logging | Axis positions | NI 9401 |
| Visualisation | Speed estimator | NI 9201 |
| | | NI 9263 |
| | | NI 9482 |

(b)

# FIG. 7

FIG. 8

FIG. 9

**FIG. 10**

FIG. 11

FIG. 12

**FIG. 13**

FIG. 14

FIG. 15

# FIG. 16

# FIG. 17

Europäisches
Patentamt

European
Patent Office

Office européen
des brevets

# EUROPEAN SEARCH REPORT

Application Number

EP 19 18 9197

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2018/219881 A1 (UNIV LEUVEN KATH [BE])<br>6 December 2018 (2018-12-06)<br>* figures 1,3,4,5 *<br>* claims 1,10 *<br>* paragraph [0002] *<br>* paragraph [0028] *<br>* paragraph [0030] *<br>* paragraph [0040] *<br>----- | 1-3,5,6,<br>10-12,14<br>4,7-9,13 | INV.<br>A61B17/34<br>A61B34/30<br>A61B90/50<br>A61F9/007<br>B25J1/00<br>B25J18/00 |
| Y | US 5 540 692 A (TIDWELL DURRELL G [US])<br>30 July 1996 (1996-07-30)<br>* column 1, line 52 - line 53 *<br>* column 2, line 55 - line 67 *<br>* column 4, line 5 - line 37 *<br>* figures 1,2,5 *<br>* claim 1 *<br>----- | 4,7-9,13 | |
| A | Jake J. Abbott ET AL: "Haptic Virtual Fixtures for Robot-Assisted Manipulation" In: "Robotics Research", 1 January 2005 (2005-01-01), Springer Berlin Heidelberg, Berlin, Heidelberg, XP055652527, ISBN: 978-3-540-48110-2 vol. 28, pages 49-64, DOI: 10.1007/978-3-540-48113-3_5, * the whole document * ----- | 11,12 | TECHNICAL FIELDS SEARCHED (IPC)<br><br>A61B<br>B25J<br>A61F |
| A | Art & Hardware: "My DIY 3D Sculpture Pantograph for under $50", Youtube, 31 January 2017 (2017-01-31), page 1 pp., XP054980050, Retrieved from the Internet: URL:https://www.youtube.com/watch?v=oE9AzV-972c [retrieved on 2019-12-16] * the whole document * ----- | 1-14 | |

The present search report has been drawn up for all claims

| Place of search | Date of completion of the search | Examiner |
|---|---|---|
| The Hague | 18 December 2019 | Seon, Jean-Antoine |

EPO FORM 1503 03.82 (P04C01)

## ANNEX TO THE EUROPEAN SEARCH REPORT
## ON EUROPEAN PATENT APPLICATION NO.

EP 19 18 9197

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2019

| Patent document cited in search report | | Publication date | Patent family member(s) | Publication date |
|---|---|---|---|---|
| WO 2018219881 | A1 | 06-12-2018 | NONE | |
| US 5540692 | A | 30-07-1996 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MOLAEI et al.** *Journal of Ophthalmic & Vision Research,* 2017, vol. 12, 212-218
- **TAYLOR.** *International Journal of Robotics Research,* 1999, vol. 18, 1201-1210
- **GIJBELS et al.** *Annals of Biomedical Engineering,* 2018, vol. 46, 1676-1685
- **EDWARDS et al.** *Nature Biomedical Engineering,* September 2018, vol. 2
- **ABBOTT et al.** Robotics Research. Springer, 2007, 49-64
- **BALICKI et al.** Medical Image Computing and Computer-Assisted Intervention MICCAI. Springer, 2009, 108-115
- **DAVIES et al.** Proceedings of the Institution of Mechanical Engineers. *Journal of Engineering in Medicine,* 1997, vol. 211, 285-292
- **LI et al.** *IEEE Transactions on Robotics,* 2007, vol. 23, 4-19
- **NASSERI et al.** 5th IEEE RAS/EMBS International Conference on Biomedical Robots and Biomechatronics. IEEE, 2014, 732-738